Europäisches Patentamt

European Patent Office

Office européen des brevets

Publication number: **0 284 315**

**A2**

# EUROPEAN PATENT APPLICATION

(21) Application number: **88302427.5**

(22) Date of filing: **18.03.88**

(51) Int. Cl.⁴: **A61M 5/14**

(30) Priority: **25.03.87 US 29583**

(43) Date of publication of application:
**28.09.88 Bulletin 88/39**

(84) Designated Contracting States:
**AT BE CH DE ES FR GB GR IT LI NL SE**

(71) Applicant: **Hsu, Shun-Fa**
**No. 20, Sheng-Te 2nd St. Chien-Chen District**
**Kaohsiung(TW)**

(72) Inventor: **Hsu, Shun-Fa**
**No. 20, Sheng-Te 2nd St. Chien-Chen District**
**Kaohsiung(TW)**

(74) Representative: **Newell, William Joseph et al**
**D. Young & Co., 10 Staple Inn**
**London WC1V 7RD(GB)**

(54) **Automatic shutting-off and alarming device for dripping injection.**

(57) An automatic shutting-off and alarming device for dripping injection use comprises a closed container (2), a buoyant cylinder (3), a magnetic member (4), a magnetically actuated contactor (5) and an alarming system. The magnetic member (4) is furnished on the bottom end of the buoyant cylinder (3) which is movably placed in the closed container (2), and the magnetically actuated contactor (5), together with the alarming system, is detachably connected to the bottom edge of the closed container (2). When injection fluid comes into the closed container (2), the buoyant cylinder (3) will float, and, when the injection fluid is almost used up, the buoyant cylinder (3) will lower down, at which the magnetic force between the buoyant cylinder (3) and the magnetically actuated contactor (5) will attract each other for shutting-off the injection fluid to stop the injection as well as to activate the alarming system for alerting medical care personnel to take proper action.

FIG . 5

# AUTOMATIC SHUTTING-OFF AND ALARMING DEVICE FOR DRIPPING INJECTION USE

An automatic shutting-off and alarming device for dripping injection use, in particular, by combining both buoyant force and magnetic attracting force to provide injection fluid with automatic shutting-off when the injection fluid is almost used up, and to alert the nurse or medical care personnel in the nursing shift center to take proper action to the patient for ensuring the injection safety, saving manpower for patient's care, saving time for subsequent injections, and promoting the hospital management.

Dripping injection fluids are general-purpose medicines utilized via blood vessel injection for patient's need. In a traditional blood vessel dripping injection equipment, the dripping injection fluid comes from a dripping bottle, through a guiding tube, a dripping nozzle and flow rate control valve, and an injection needle, then goes into the blood vessel of a patient. This traditional blood vessel dripping injection fluid and equipment has been utilized for a long time by doctors for patient treatment or patient body strength and spirits maintenance, though it works well, but still has shortcomings as follows:

1. Since the dripping injection fluid is pumped into the blood vessel by atmospheric pressure and gravitational force, therefore, in order to maintain patient's safety and to prevent accident, a patient under dripping injection treatment is closedly watched by other dedicated person. Furthermore, a bottle of dripping injection usually requires several hours to finish the injection, also one bottle after another bottle for subsequent injections are commonly required; therefore, dripping injection becomes a tiresome and time-consuming work.

2. As time goes by, in a busy business and industrial age, spending a lot of time to accompany a patient becomes an expensive burden.

3. Because traditional dripping injection is taken care by people who accompanies the patient, therefore, accident may happen due to sleep or neglect.

4. When dripping injection fluid is almost used up and the fluid level lowers down under the dripping valve, if subsequent injection is continuous, the trapped air shall exist between the old injection fluid and the new injection fluid. Under this condition, drip injection cannot be directly connected to continue until a new set of dripping injection equipment is changed. In this manner, the patient cannot but suffer piercing pain once again.

5. Though electronic surveillance equipments for dripping injection are available nowaday, they are short of automatic stop devices. In addition, not only they are complex in structure, power consuming, limited by power source requirement, expensive, high out of order rate and difficult in maintenance, but also they have no any contactors for being used to alert the nurse or medical care personnel in the nursing shift center.

Based on these findings, the inventor provides a new automatic shutting-off and alarming device for dripping injection use. This new device is not only a desirable device to remove all the above-mentioned shortcomings, it is also a safe, practical, reliable, time saving and economical device to be widely adapted for medical applications.

The main purpose of the present invention is to provide an automatic shutting-off and alarming device for dripping injection use, in particular, which comprises a closed container, a buoyant cylinder, a ring magnet, a pivotally clamped magnetically-actuated contactor and an alarming system. This device can alert the medical personnel to take immediate action for a patient when the dripping injection fluid is almost used up, and it is also advantageous for the promotion of management of a hospital, and these advantages constitute a specific feature of the present invention.

The other purpose of the present invention is to provide an automatic shutting-off and alarming device for dripping injection use, of which, the buoyant cylinder will lower down to shut off the injection fluid for securing injection safety when the injection fluid is almost used up. This advantage constitutes another specific feature of the present invention.

Another object of the present invention is to provide an automatic shutting-off and alarming device for dripping injection use, of which, when the buoyant cylinder is lowered down to shut off the injection fluid, the magnetic force between the ring magnet on a bottom side of the buoyant cylinder and the pivotally clamped magnets of the magnetically-actuated contactor will, at the same time, activate two alarming signals, one for the patient's bedroom and the other for the nursing shift center so as to alert the medical personnel to take proper action for the patient and save the nursing manpower and time. This advantage also constitutes a specific feature of the present invention.

Still another purpose of the present invention is to provide an automatic shutting-off and alarming device for dripping injection use, of which the center portion on a bottom side of the buoyant cylinder is forced into a conical shape. This conical shape structure being incorporated with an outlet port of the closed container constitutes a shutting-off valve, which will be actuated by both the gravitational and the magnetic forces to shut off the injection fluid when the fluid is almost used up. In addition, the valve can also prevent the patient's blood from reversely flowing into the dripping injection tube in case the blood pressure of the patient is increased abruptly at this time. This advantage also constitutes one of the specific features of the present invention.

One further object of the present invention is to provide an automatic shutting-off and alarming device for dripping injection use, of which, when the injection fluid is shut off upon being almost used up, both the closed container and the tube lines of the dripping injection equipment will maintain enough injection fluid, and subsequent injection fluid bottle can be directly connected to it without considering any trapped air to be injected into the patient's vessel. This advantage also constitutes one of the specific features of the present invention.

The structure, function, and characteristics of the present invention are described in detail with reference to the attached drawings, in which:

Figure 1 shows the perspective view of the automatic shutting-off and alarming device for dripping injection use of the present invention;

Figure 2 shows the sectional and perspective view of the closed container and the buoyant cylinder for the automatic shutting-off and alarming device for dripping injection use of the present invention;

Figure 3 shows the sectional and perspective view of a magnetically-actuated contactor for the automatic shutting-off and alarming device for dripping injection use of the present invention;

Figure 4 shows the implementation diagram of the automatic shutting-off and alarming device for dripping injection use of the present invention;

Figure 5 shows the sectional view of the automatic shutting-off and alarming device of the present invention with functions illustrated;

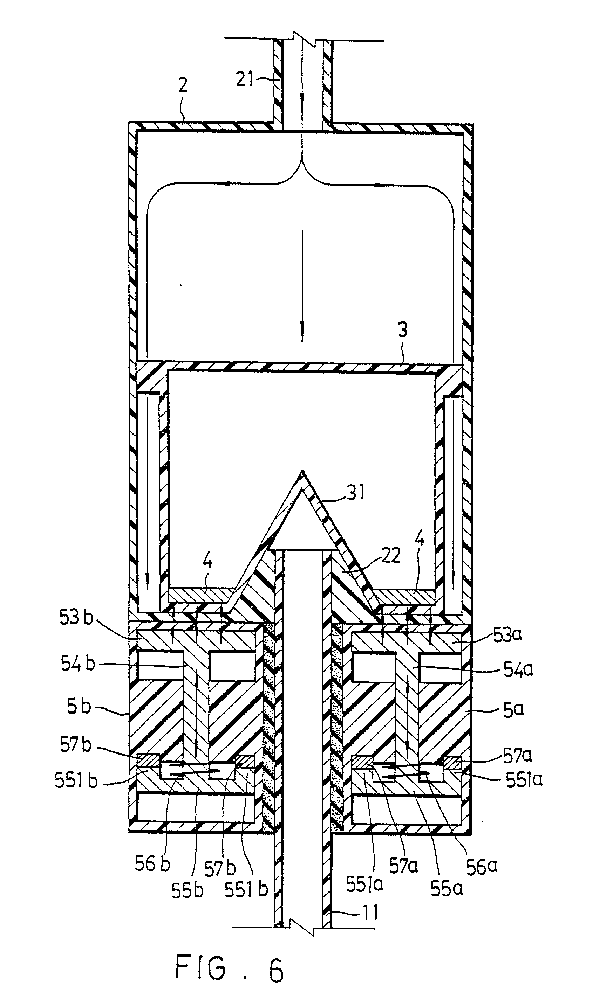

Figure 6 shows the sectional view of the automatic shutting-off and alarming device for dripping injection use of the present invention with the shutted-off position illustrated;

Figure 7 shows the circuit diagram for the embodiment example of the alarming system of the automatic shutting-off and alarming device for injection use of the present invention; wherein contactor 57b, 551b shall be connected to the hospital existing equipments, which are shown in dotted lines, such as emergency push button connected to the nursing shift center or any other medical instruments;

Figure 8 shows another embodiment example of the magnetically-actuated contactor for alarming device of the automatic shutting-off and alarming device for dripping injection use of the present invention;

Figure 9 is a perspective and exploded view of an alternative embodiment of the automatic shutting-off and alarming device for dripping injection use of the present invention;

Figure 10 is a bottom view of the embodiment shown in Fig. 9; and

Figure 11 is a vertical sectional view of the assembled embodiment of Fig. 9.

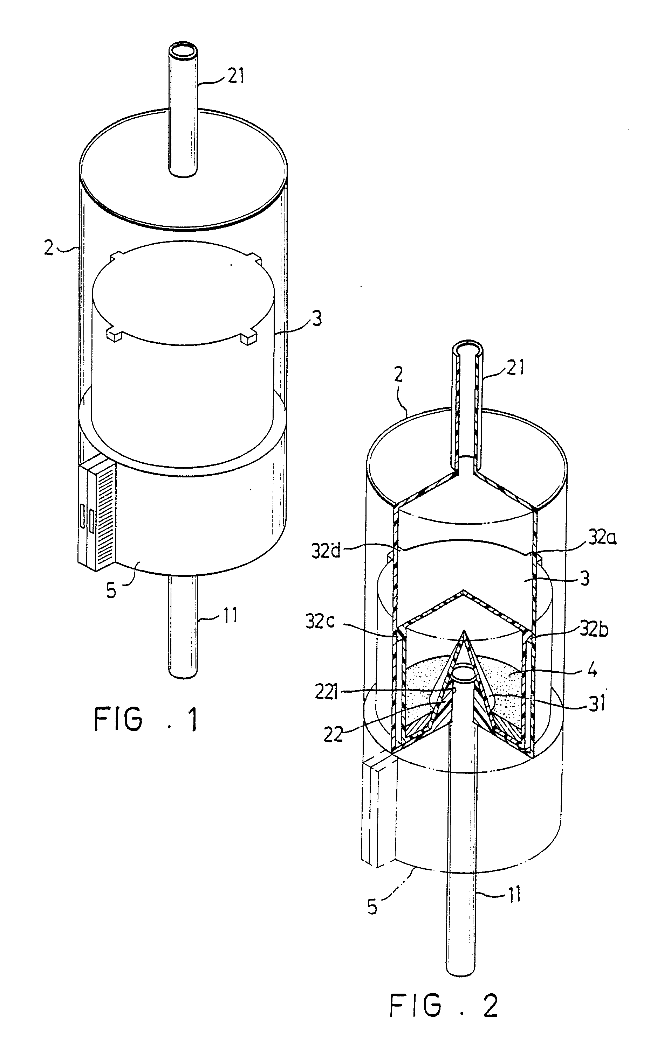

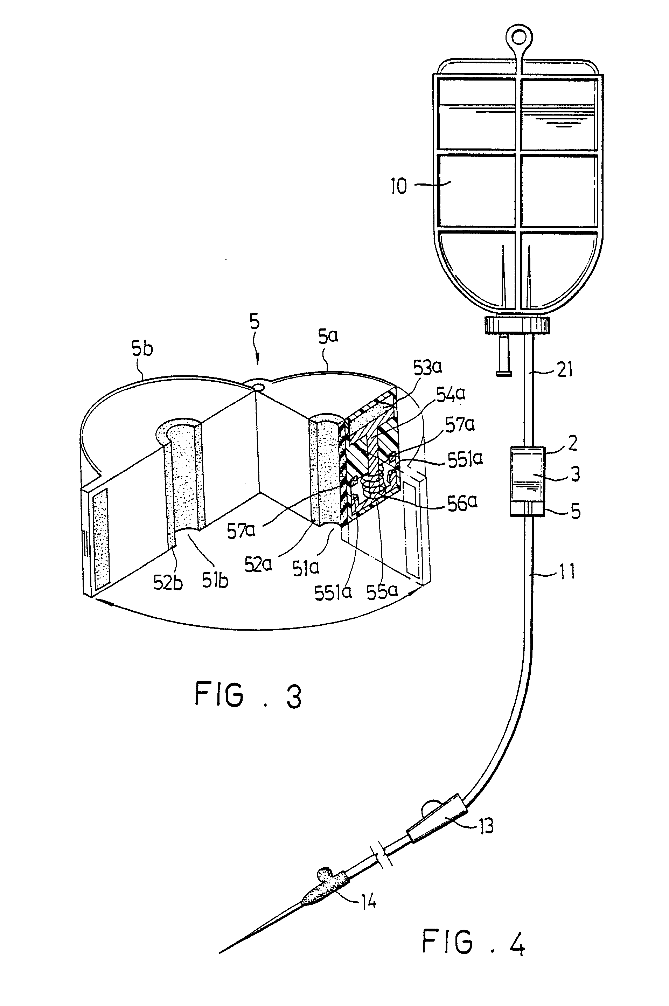

Referring to Figs. 1 to 3, the automatic shutting-off and alarming device for dripping injection use of the present invention comprises a closed container 2, a buoyant cylinder 3, a ring magnet 4, a magnetically-actuated contactor 5, and an alarming device, in which the closed container 2 is made of transparent, PE plastic material, and has an inlet tube 21 on its top end for connecting a needle, which is inserted into the injection fluid bottle, and a cone-shaped outlet port 22 and a socket 221 on its bottom end for plugging in an injection fluid output tube. The buoyant cylinder 3 with multiple guiding ears 32a, 32b, 32c and 32d is placed into the closed container 2; the buoyant cylinder 3 has a ring magnet 4 which is fixed onto its bottom end, and on the same bottom end it is integratedly moulded with the buoyant cylinder body 3 with a conical portion 31, and this conical portion can fit water tightly with the conical port 22 of the closed container 2.

The magnetically actuated contactor 5 comprises two symmetrical contactor bodies 5a, 5b, and an integrated magnetically actuated contactor 5 is assembled by clamping these two contactor bodies together. The inner walls around the two central half-openings 51a and 51b are furnished with rubber layers 52a and 52b which are utilized to tighten the injection fluid out-flowing tube when the magnetically-actuated contactor bodies 5a, 5b are clamped together. Two semi-ring magnets 53a and 53b are respectively furnished on the upper ends of each of the contactor bodies 5a and 5b, and the bottom ends of these semi-ring magnets are attached respectively to their connecting rods 54a and 54b of which the bottom ends are attached to their respective movable arms 55a and 55b, and two coiled springs 56a and 56b are furnished respectively under each of movable arms 55a and 55b. Also, contact points 551a, 551b are furnished on top sides of movable arms 55a, 55b, and corresponding contact points 57a, 57b are furnished on the bottom side of the contactor bodies as they are facing the contacting points 551a, 551b on movable arms 55a and 55b. These structural components form an integrated magnetically-actuated contactor. Furthermore, the polarities on top sides of each of the semi-ring magnets 53a, 53b of the magnetically-actuated contactor are just the reversal to the polarities on the bottom side of the ring magnet 4 located on the bottom end of the buoyant cylinder. Therefore, attractive force is activated between them when injection fluid is almost used up, which in turn activates the contact points 57a and 57b to produce alarming signals.

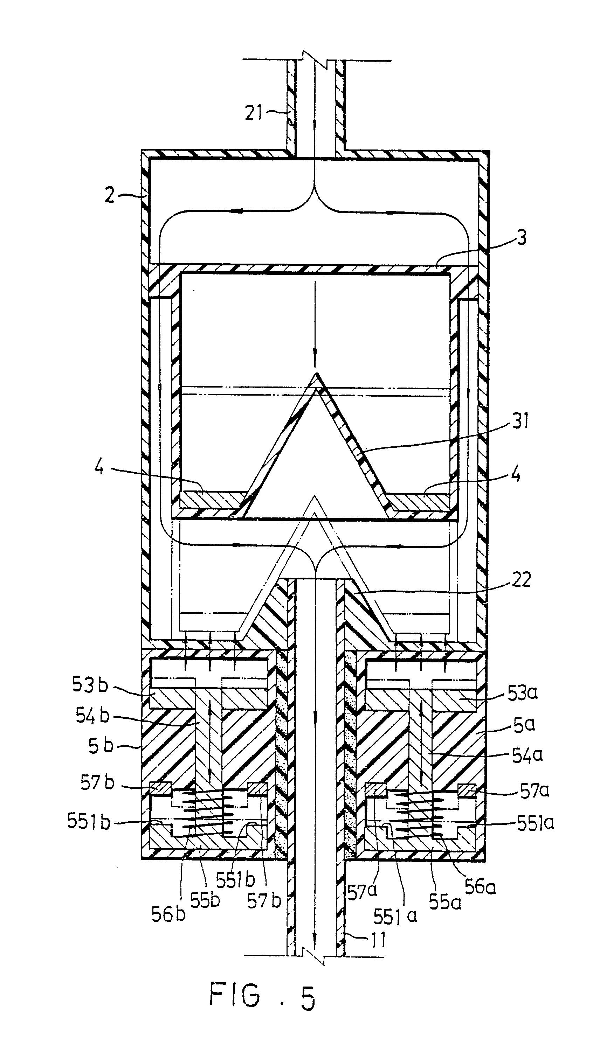

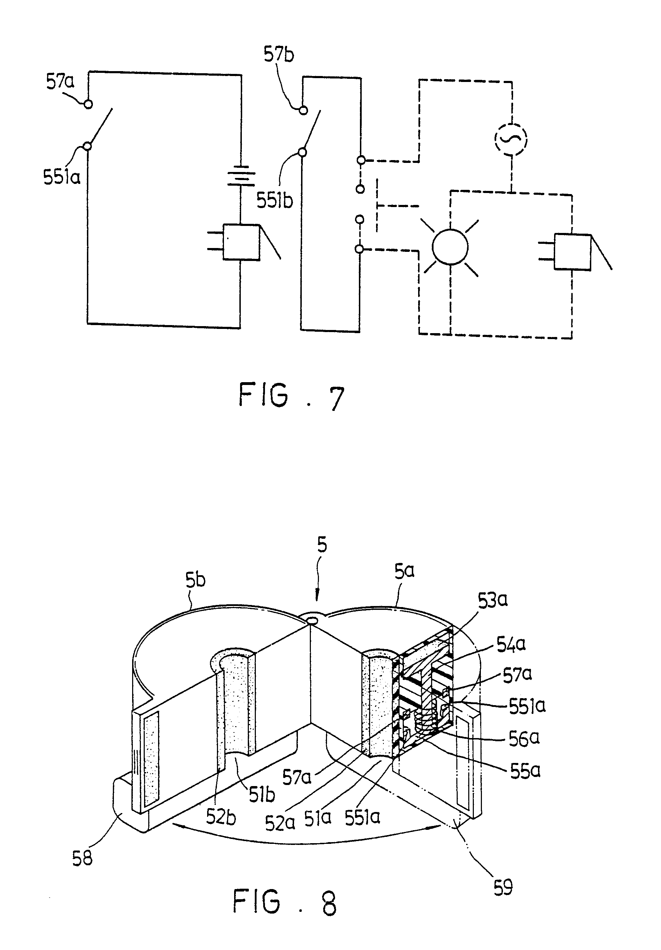

Referring to Fig. 4, the automatic shutting-off and alarming device for dripping injection use of the present invention utilizes the guiding tube 21 on top of the closed container 2 to connect the injection fluid bottle 10 via a needle. When injection fluid comes into the closed container 2, a buoyant force will cause the buoyant cylinder 3 to float upwardly. In addition, an injection guiding tube 11 is inserted into the fluid outlet socket 221 to lead the injection fluid into the flow rate control switch 13, needle 14, and then goes into the blood vessel of a patient. Since magnetically actuated contactor 5 is clamped to the injection fluid guiding tube 11 at the bottom end of the closed container 2, at this time, the gravitational force plus the spring force of coiled spring 56a, 56b acting on semi-ring magnets 53a and 53b, connecting rods 54a and 54b, and movable arms 55a and 55b, will cause them to fall downwardly to break off the connection of each pair of contact points 551a, 57a and 551b, 57b (as shown in Fig. 5). When the injection fluid is almost used up, the injection fluid level in the closed container will gradually lower down to a certain level, and so does the buoyant cylinder 3. At this time, the distance between the ring magnet 4 and the semi-ring magnets 53a and 53b reaches its shortest; thus, a maximum attracting force is activated between them, which pulls down instantly the conical portion 31 on the bottom end of the buoyant cylinder 3 over the conical outlet port 22 of the closed container 2 to shut off the fluid from flowing into the socket 221 for stopping the dripping injection in one way, and pulls up instantly the contact points 551a, 551b on movable arms 55a and 55b to connect points 57a and 57b on the bottom side of the contactor bodies 5a, 5b via semi-ring magnets 53a and 53b, connecting rods 54a and 54b, and movable arms 55a and 55b in another way to activate the alarming device (as shown in Fig.7) for issuing two alarm signals, one for patient's bedroom and the other for nursing shift center, to alert the medical personnel to take proper action for the patient. At this time, if subsequent injection is not required, the only thing to do is the detachment of the magnetically actuated contactor 5. Once the magnetically-actuated contactor 5 is detached from the bottom end of the closed container, no more strong magnetic attracting force is exerted on the movable arms 55a and 55b. Since the movable arms 55a and 55b are connected to the semi-ring magnets 53a and 53b on top side of the magnetically-actuated contactor bodies 5a and 5b via the connecting rods 54a and 54b. the gravitational force plus the spring force of the coiled spring 56a and 36b will pull down the movable arms 55a and 55b and break the connection between contact point pairs 551a, 57a and 551b, 57b. and the alarming signals are therefore stopped; then, remove the needle from the blood vessel of the patient to complete the dripping injection process. If subsequent dripping injection is required, what the medical care personnel is going to do then is to remove the guiding tube needle from the empty injection fluid bottle, and inserts it into a new injection fluid bottle, and, when the injection fluid coming into the closed container reaches a certain level, detaches the magnetically-actuated contactor 5 from the bottom end of the closed container 2 to open the injection fluid flowing passage for starting the subsequent dripping injection. After buoyant cylinder 3 has risen up from the closing-down position, then attach the magnetically-actuated contactor 5 again to the bottom end of the closed container so as to recover its alarming function. In this manner, because there is no any trapped air exists between the old dripping injection fluid and the new dripping injection fluid, so the injection needle needs not to be pulled out from the blood vessel for removing the trapped air and inserting it back again. Furthermore, a battery compartment 58 and a buzzer 59 can be furnished on the bottom side of the magnetically-actuated contactor 5 (as shown in Fig. 8). In this way, the present invention possesses a self-powered alarming system.

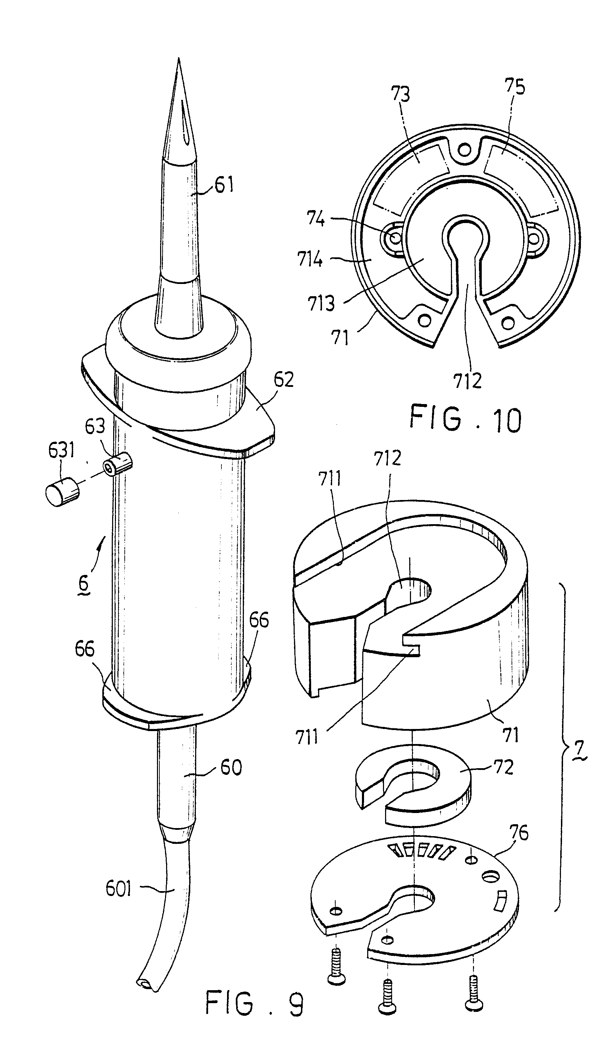

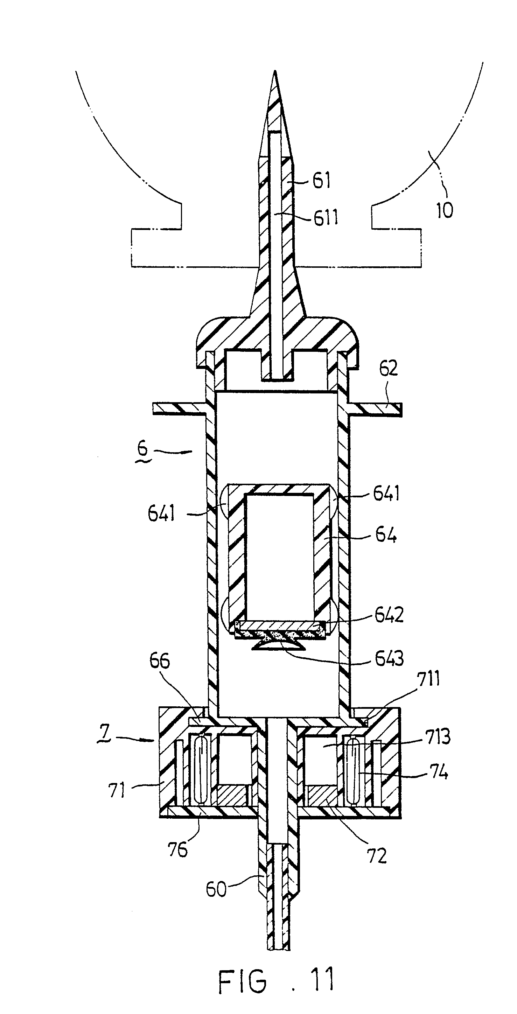

Referring to Figs 9 to 11, there is shown an alternative embodiment of the automatic shutting-off and alarming device for dripping injection use according to this invention. This embodiment also comprises two main components, a closed container 6 and a magnetically-actuated contactor 7. A hollow piercing rod 61 having an inner passageway 611 formed therein is furnished at a top end of the closed container 6 for being inserted into an injection fluid bottle 10 so as to guide the injection fluid into the closed container 6 through the hollow tip of the piercing rod 61. A flanged fin 62 is located around an upper end portion of the closed container 6 for facilitating either the piercing through or the pulling out of the piercing rod 61. In addition, before processing a drip injection, the injection fluid is guided into the closed container 6 by keeping air duct 63 open so as to enable the trapped air to escape from the inner section of the closed container 6 and the injection fluid 10 to flow into the closed container 6, then, the air duct 63 is closed up with the duct cover 631 after the buoyant cylinder 64 floats to a determined position in the closed container 6. The buoyant cylinder 64, as shown in Fig. 11, is also movably installed in the closed container 6, and a plurality of guiding ears 641 are formed on the outer surface of the buoyant cylinder 64 for guiding its up-and-down movement in the closed container 6 along with the rising and falling conditions of the injection fluid. A magnetic member 642, such as a ferrite or a magnet, is disposed at the lower end of the buoyant cylinder 64, and a plugging member 643, which is formed in a reverse funnel shape, is furnished on the bottom side of the magnetic member 642 for shutting off the fluid guiding tube 60, which is located at the lower end of the closed container 6 for leading the injection fluid out of the closed container, when the injection fluid is almost used up and the buoyant cylinder 64 is lowered down.

The above described embodiment, as shown in Figs. 9-11, resides in the combination of the magnetically actuated contactor 7 and the closed container 6 as well as the structure of the magnetically actuated contactor 7; wherein, the closed container 6 has at least a coupling flange 66 extending at the lower edge, and the magnetically actuated contactor 7 comprises a contacting body 71 having an open groove 712 vertically formed in one side and a scarfing slot 711 horizontally located at the upper end. In assembling the embodiment, placing the soft output tube 601, which is connected to the fluid guiding tube 60 at one end, in the open groove 712 of the magnetically actuated contactor 7, pushing the magnetically actuated contactor 7 upward so as to insert the fluid guiding tube 60 in the open groove 712, and then, turning either the closed container 6 or the magnetically actuated contactor 7 for closely engaging the coupling flange 66 with the scarfing slot 711, the magnetically actuated contactor 7 will be tightly attacted to the lower end of the closed container 6.

As shown in Figs. 9 and 10, in addition to the open groove 712 and the scarfing slot 712 in the contacting body 71, the magnetically actuated contactor 7 further comprises: an accommodating chamber 713 formed in the center around the open groove 712; a horse-shoe shaped magnet 72 movably disposed in the accommodating chamber 713; a receiving recess 714 formed around the periphery of the accommodating chamber 713; an alarming device composed of a battery circuit 73, a reed switch 74 and an alarming signal generator 75, which are respectively disposed in the receiving recess 714 and electrically connected with each other, furnished in the receiving recess 714 of the magnetically actuated contactor 7 for being actuated to generate alarming signals through the reed switch 74; and a covering member 76 formed in conjunction with the contacting body 71 being fixed on the bottom end of the contacting body 71.

It shall be appreciated that since the structure and electrical connection of the battery circuit, reed switch and alarming signal generator are well known to those skilled in the art. detailed description and illustration of the alarming device is therefore omitted for brevity. In addition, the alarming signal generator 75 can be a buzzer and also can be electrically connected to a monitoring system of the nursing shift center.

As shown in Fig. 11, the reed switch 74 situated in the receiving recess 714 is close to the magnet 72 in the accommodating chamber 713. When the buoyant cylinder 64 is located at the upper portion of the closed container 6 with the injection fluid, and the magnet 72 is positioned at the lower surface of the accommodating chamber 713, the reed switch 74 is at an open state, and no alarming signal is generated by the alarming device; however, when the buoyant cylinder 64 moves down along with the falling of the injection fluid in the closed container 6, magnetic attractive force produced between the magnetic member 642 and the magnet 72 will become stronger and stronger until the buoyant cylinder 64 reaches a certain level in the closed container 6. In this condition, the magnet 72 will be pulled upward, and the reed switch 74 is therefore closed up through the attraction of the magnet 72, and the alarming signal generator 75 is therefore activated to effect alarming function thereat. In the meantime, the plugging member 643 will also be pulled downward to shut off the fluid guiding tube 60 and stop the dripping injection.

## Claims

1. An automatic shutting-off and alarming device for dripping injection use, which comprises:

a closed container (2), which is an empty body made of transparent material, the top end of the empty body being furnished with a fluid guiding tube (21) for connection to a bottle (10) containing dripping injection fluid, the bottom end of the empty body being furnished with an injection fluid outlet socket (221) for plugging in an injection fluid guiding tube (11);

a buoyant cylinder (3), which is installed into the closed container (2) to provide buoyant force;

a ring magnet (4), which is attached to the bottom end of the buoyant cylinder (3);

a magnetically-actuated contactor (5), which comprises two symmetrical contactor bodies (5a, 5b), pivotally connected and clamped together as an integrated unit, the central portion of these symmetrical contactor bodies (5a, 5b) being furnished respectively with a semi-ring tightening bore (51a, 51b), the top side of each symmetrical contactor body (5a, 5b) being furnished with a semi-ring magnet (53a, 53b), a movable arm (55a, 55b), having on each top side a contact point (551a, 551b), being attached firmly to each of the semi-ring magnet (53a, 53b) via a connecting rod (54a, 54b), a coiled spring (56a, 56b) furnished under the bottom side of each movable arm (55a, 55b), two contact points (57a, 57b), one on each side, just opposite to and matching with the contact points (551a, 551b), being furnished on the bottom surface at the intermediate section of the magnetically-actuated contactor (5); with the above-mentioned structural components, and the magnetically-actuated contactor (5) attached to the bottom end of the closed container (2) by clamping it at the injection fluid guiding tube (11) below the closed container (2), the dripping injection being able to be automatically shut off by strong magnetic attracting force between the ring magnet (4) on the bottom end of the buoyant cylinder (3) and the semi-ring magnets (53a, 53b) on top side of the magnetically actuated contactor (5) when the buoyant cylinder (3) is lowered down to a certain level at which the injection fluid is almost used up as well as the same magnetic attracting force pulling the movable arms 55a, 55b) up to close the contact points (57a, 551a; 57b, 551b)and producing two alarming signals, one for patient's bedroom and the other for nursing shift center, to alert the medical care personnel to take immediate action for the patient.

2. An automatically shutting-off and alarming device for dripping injection use as set forth in claim 1, in which, the polarities between the ring magnet (4) on the bottom end of the buoyant cylinder (3) and the semi-ring magnets (53a, 53b) of the contactor bodies (5a, 5b) are reversal to each other.

3. An automatic shutting-off and alarming device for dripping injection use as set forth in claim 1, in which, the outlet port on an inner bottom side of the closed container (2) and the bottom side of the buoyant cylinder (3) are formed into a conical shape (22, 31) for firmly shutting-off the injection fluid when the dripping injection fluid is almost used up.

4. An automatic shutting-off and alarming device for dripping injection use, as claimed in claim 1, wherein the automatic shutting-off and alarm performances are carried out by way of said buoyant object combined with magnetic force at the impending termination of the injection process.

5. An automatic shutting-off and alarming device for dripping injection use, which comprises:

a closed container (6) having a top end furnished with a piercing means (61) for guiding injection fluid into the closed container (6) and a lower end provided with a fluid guiding tube (60) for leading the injection fluid out of the closed container (6) in effecting dripping injection;

a buoyant cylinder (64) movably disposed in the closed container (6) for moving up and down along with the rising and falling of the injection fluid in the closed container (6);

a magnetic member (642) furnished at a lower end of the buoyant cylinder (64) for producing magnetic attracting force therefrom;

a plugging member (643) disposed at a bottom side of the magnetic member (642) for shutting off the fluid guiding tube of the closed container (6) along with the lowering down of the buoyant cylinder (64);

a magnetically actuated contactor (7) having an open groove (712) vertically formed in one side detachably connected to the lower end of the closed container (6) with the fluid guiding tube (60) thereof being situated in the open groove (712);

a magnet (72) movably installed in the magnetically actuated contactor (7) for being attracted up by the attracting force of the magnetic member (642) along with the lowering down of the buoyant cylinder (64);

a reed switch (74) provided in the magnetically actuated contactor (7) in conjunction with the magnet (642) of the buoyant cylinder (64) for being actuated to close along with the rising up of the magnet (72);

a battery circuit (73) disposed in the magnetically actuated contactor (7) and electrically connected to the reed switch (74); and

an alarming signal generating means (75) installed in the magnetically actuated contactor (7) and electrically connected with the reed switch (774) for generating alarming signals therefrom; so that when the buoyant cylinder (64) is lowered down to a certain level at which the injection fluid is almost used up, and the magnetic attracting force of the magnetic member (642) pulls up the magnet (72) in the magnetically actuated contactor (7), the reed switch (74) will be closed to producing alarming signals for alerting medical care personnel, and the plugging member (643) of the buoyant cylinder (64) will be pulled down at the same time to shut off the fluid guiding tube (60) of the closed container (6) for stopping the injection.

6. An automatic shutting-off and alarming device for dripping injection use as set forth in claim 5 wherein said closed container (6) includes at least a coupling flange (66) extending at a lower edge, and said magnetically actuated contactor (7) comprises a scarfing slot (711) formed at an upper end; so that, by inserting the coupling flange (66) into the scarfing slot (711), the magnetically actuated contactor (7) will be tightly engaged with the lower end of the closed container (6).

7. An automatic shutting-off and alarming device for dripping injection use as set forth in claim 6 wherein said magnetically actuated contactor (7) further comprises an accommodating chamber (713) formed around the open groove (712) for allowing the up-and-down movement of the magnet (72) therein.

8. An automatic shutting-off and alarming device for dripping injection use as set forth in claim 7 wherein said magnetically actuated contactor (7) further comprises a receiving recess (714) formed around the periphery of the accommodating chamber (713) for the installation of the reed switch (74), the battery circuit (73) and the alarming signal generating means (75), and a covering member (76) connected to a bottom end of the magnetically actuated contactor (7) over the receiving recess (714).

9. An automatic shutting-off and alarming device for dripping injection use as set forth in claim 5 wherein said plugging member (643) is formed and positioned in a reverse funnel shape.

10. An automatic shutting-off and alarming device for dripping injection use as set forth in claim 5 wherein said buoyant cylinder (64) further comprises a plurality of guiding ears (641) separately formed on an outer surface thereof for guiding the up-and-down movement of the buoyant cylinder (64) along with the rising and falling of the injection fluid in the closed container (6).

11. An automatic shutting-off and alarm device for a drip feed, comprising:-

a chamber (2) having an inlet (21) and an outlet (221) in opposed inlet and outlet end regions thereof,

a bouyancy member (3) movably disposed in said chamber (2) and movable in use under gravity towards said outlet end region to a position in which it substantially closes said outlet (221), and means (4, 53a, 53b) for magnetically attracting the buoyancy member towards the outlet end region,

whereby in use when the volume of fluid within said chamber is greater that a predetermined volume, the buoyancy member (3) floats in said fluid, spaced from said outlet (221), but that when the volume of fluid reaches or approaches said predetermined volume, the forces due to gravity and magnetic attraction overcome the buoyancy force so that the buoyancy member (3) is drawn down substantially to close said outlet (221).

FIG . 1

FIG . 2

FIG . 3

FIG . 4

FIG . 5

FIG . 6

FIG . 7

FIG . 8

61

62

63

631

6

66

66

66

60

601

FIG . 9

73    75

74

714

713

71

712

FIG . 10

711    712

711

71

72

7

76

FIG . 11